# EUROPEAN PATENT APPLICATION

(11) **EP 3 708 142 A1**
(43) Date of publication of application: **16.09.2020**
(21) Application number: 18876023.5
(22) Date of filing: 30.10.2018
(51) Int. Cl.: A61K 8/06, A61K 8/25, A61K 8/89, A61K 9/127, A61K 47/04, A61Q 1/00, A61Q 19/00, B01J 13/00

(54) **WATER-IN-OIL EMULSION COMPOSITION INCLUDING HYDROPHOBIC SPHERICAL SOL-GEL SILICA FINE PARTICLES**

(30) Priority: 07.11.2017 JP 2017214820
(71) Applicant: Shin-Etsu Chemical Co., Ltd., Tokyo 100-0004 (JP)
(72) Inventor: Oki Takahito, Annaka-shi Gunma 379-0224 (JP); MATSUMURA Kazuyuki, Annaka-shi Gunma 379-0224 (JP); KIMURA Tsuneo, Annaka-shi Gunma 379-0224 (JP)
(74) Representative: V.O.
(86) International application number: PCT/JP2018/040378
(87) International publication number: WO 2019/093199

(57) **Abstract**

One of objects of the present invention is to provide a water-in-oil emulsion composition useful for cosmetics and skin preparations, which is excellent in storage stability without any surfactant and is also excellent in safety. The present invention provides a water-in-oil emulsion composition comprising
(a) an oily substance in an amount of 100 parts by mass,
(b) water, or water and a water-soluble substance in an amount of 11 to 800 parts by mass, and
(c) hydrophobic, spherical sol-gel silica fine particles having a volumetric median diameter (D50) of 5 to 1500 nm in an amount of 0.5 to 40 parts by mass.

## Description

### Technical Field

The present invention relates to a water-in-oil Pickering emulsion composition comprising hydrophobic, spherical sol-gel silica fine particles which function as surfactants. In particular, the present invention relates to emulsion compositions which are suitable in the field of cosmetics and skin preparations.

### Background of the invention

An emulsion comprises two phases which are immiscible or little missile with each other. One phase comprises fine liquid particles which are dispersed in another continuous phase.

An emulsion in which oil droplets are finely dispersed in water is called an oil-in-water emulsion (O/W type emulsion). An emulsion in which water droplets are finely dispersed in oil is called a water-in-oil emulsion (W/O type emulsion). The relation between the two phases in the oil-in-water emulsion and the water-in-oil emulsion is opposite. The basic properties are determined by a type of oil.

Emulsions generally require a surfactant. The surfactant has a polar (hydrophilic) moiety and a nonpolar (lipophilic) moiety and, thus, is amphiphilic to the two phases. Surfactants are classified into ionic (anionic, cationic, and amphoteric/zwitterionic) and nonionic types, depending on the property of the hydrophilic part.

When water and oil are mixed, an interface tension arises usually at an oil/water interface to make the interface area as smaller as possible. Then, oil and water segregate from each other to form a stable state, rather than a dispersion whose an area of an interface is larger. However, when a surfactant is present, the surfactant molecules adsorb on the oil/water interface to lower, the surface tension, so that the dispersion is stable even though its interface area is larger. That is, the surfactant stabilizes an emulsion.

As described above, a surfactant is one of the important factors to form a stable emulsion which does not cause phase separate.

Generally, use of surfactants in cosmetics or skin preparations is itself accepted. Nevertheless, surfactants, like other chemical materials, may cause an allergic reaction under certain circumstances or reactions based on the user's hypersensitivity. Therefore, it is required for safety to reduce an amount of the surfactant as small as possible or avoid use of a surfactant at all.

Around 1900 year, Pickering discovered an emulsion stabilized by solid fine particles adsorbing on a liquid/liquid interface and, prepared a paraffin/water emulsion which was stabilized by the various solid fine particles, such as basic copper sulfate and iron sulfate, or other metal sulfate salts. Emulsions prepared using such solid fine particles are commonly called "Pickering emulsion". Thus, emulsions can be prepared without a surfactant.

In an emulsion prepared using solid fine particles, the fine solid particles have higher adsorption energy than conventional surfactant molecules. Once the fine solid particles having an appropriate wettability adsorb on the interface, the particles do not easily desorb the interface. Therefore, the emulsion formed is stabile. It is known that the Pickering emulsion causes less coalescence of the droplets, compared to the conventional emulsions surfactant.

In recent years, art of using Pickering emulsions as bases for cosmetics or skin preparations has been developed and a number of researches have been reported, such as Non-Patent Literature 1. The use of Pickering emulsions in the field of perfumery cosmetics has also been proposed.

For example, Patent Literature 1 describes an oil-in-water emulsion composition comprising hydrophobic fine particles which is useful for cosmetics and skin preparations. Patent Literature 2 describes an oil-in-water emulsion composition which has excellent emulsifying stability and comprises solid fine particles such as hydrophobic silica, hydrophobic cellulose, silicone resin powder, hollow hemispherical silicone particles, polyamide resin, talc and hydrophobic pigment. Patent Literature 3 describes a water-in-oil emulsion composition comprising a thermal decomposed silica (dry silica) subjected to a surface treatment or polysilsesquioxane particles obtained by polymerizing an alkylalkoxysilane.

### Prior Art Literatures

### Patent Literatures

Patent Literature 1:
   Japanese National Phase Publication No. 2016-540783
Patent Literature 2:
   Japanese Patent Application Laid-Open No. 2010-59076
Patent Literature 3:
   Japanese Patent No. 2656226

### Non-Patent Literature

Non-Patent Literature 1:
B. Binks et al., Advances in Colloid and Interface Science 2003, 100-102

### Summary of the Invention

### Problems to be Solved by the Invention

However, the water-in-oil Pickering emulsions described in the Patent Literatures have low storage stability to cause phase separation and, therefore, are insufficient for applications in cosmetic products such as creams.

Accordingly, one of objects of the present invention is to provide a water-in-oil emulsion composition useful in cosmetics and skin preparations, which is excellent in storage stability even without a surfactant and is also excellent in safety.

### Means to Solve the Problems

As a result of intensive studies to solve the afore-mentioned problems, the inventors have found that a water-in-oil emulsion which comprises, as a surface-active material, hydrophobic, spherical sol-gel silica fine particles and are obtained by hydrophobizing a surface of hydrophilic, spherical sol-gel silica fine particles which have a uniform particle diameter, (*i.e.,* a narrow particle size distribution) and a low agglomeration property, has excellent strage stability even without a conventional surfactant.

That is, the present invention provides a water-in-oil emulsion composition comprising
(a) an oily substance in an amount of 100 parts by mass,
(b) water, or water and a water-soluble substance in an amount of 11 to 800 parts by mass, and
(c) hydrophobic, spherical sol-gel silica fine particles having a volumetric median diameter (D50) of 5 to 1500nm in an amount of 0.5 to 40 parts by mass.

### Effect of the Invention

The water-in-oil emulsion composition of the present invention has excellent storage stability and high safety. Thus, the present emulsion composition may be used as a stable and safe material has the functions comparable with those of a water-in-oil emulsion containing a surfactant, and is suitable for cosmetics and skin preparations.

### Brief Explanation of the Drawings

Figure 1: a polarizing microscope image of the emulsion composition prepared in Example 4.
Figure 2: a polarizing microscope image of the emulsion composition prepared in Example 10.
Figure 3: an overall view of the particles in an electron microscope image at the oil/water interface of a silica fine particle in the present emulsion composition.
Figure 4: a partial view of the particles in an electron microscope image of silica fine particles at the oil/water interface in the present emulsion composition.

### Detailed Description of the Invention

The present invention will be described below in detail.

The emulsion composition of the present invention is stabilized by solid fine particles which adsorb on the liquid/liquid interface and is a so-called Pickering emulsion. Thus, the present emulsion composition preferably contains no surfactant such as an emulsifier.

### Oily phase

Examples of the oily substance include non-polar oils such as a silicone oil and a hydrocarbon oil; polar oils such as an aliphatic alcohol and an ester; and a mixture of them. The non-polar oil is preferred.

Examples of the silicone oil include a linear or branched organopolysiloxane having a low viscosity to a high viscosity, such as dimethylpolysiloxane, tristrimethylsiloxymethylsilane, caprylylmethicone, phenyltrimethicone, tetrakis(trimethylsiloxy)silane, methylphenylpolysiloxane, methylhexylpolysiloxane, methylhydrogenpolysiloxane and a dimethylsiloxane/methylphenylsiloxane copolymer; cyclic organopolysiloxanes such as octamethylcyclotetrasiloxane, decamethylcyclopentasiloxane, dodecamethylcyclohexasiloxane, tetramethyltetrahydrogencyclotetrasiloxane and tetramethyltetraphenylcyclotetrasiloxane; silicone rubbers such as a gummy dimethylsiloxane/methylphenylsiloxane copolymer, and cyclic organopolysiloxane solutions of a silicone gum and rubber, an alkyl modified silicone, a long chain alkyl modified silicone, and a silicone resin and a dissolved material of a silicone resin. Preferably, a linear or branched organopolysiloxane having a low viscosity to a high viscosity, such as dimethylpolysiloxane, phenyltrimethicone, methylphenylpolysiloxane, methylhexylpolysiloxane, methylhydrogenpolysiloxane, dimethylsiloxane/methylphenylsiloxane copolymers, and cyclic organopolysiloxanes such as octamethylcyclotetrasiloxane.

The silicone oil may be organically modified. The organically modified silicone oil has one or more organic functional groups in the structure of the silicone oil. Examples of the organically modified silicone oil include modified silicone oils such as an amino-modified silicone oil, an epoxy-modified silicone oil, a polyether-modified silicone oil, a carboxy-modified silicone oil, an alcohol-modified silicone oil, an alkyl-modified silicone oil, an ammonium salt-modified silicone oil and a fluorine-modified silicone oil.

Examples of the hydrocarbon oil include a lower alkane having 6 to 16 carbon atoms which may be linear, branched or cyclic, such as hexane, undecane, dodecane tridecane and isoparaffin. Alternatively, it may be a linear or branched hydrocarbon having 17 or more carbon atoms, such as a liquid paraffin, a liquid petrolatum, a polydecene, a hydrogenated polyisobutene, olive oil, sesame oil, rice bran oil, safflower oil, soybean oil, corn oil, rapeseed oil, palm oil, palm kernel oil, sunflower oil, almond oil, cottonseed oil, cotton oil, peanut oil, fish oil, squalene and squalane.

Aliphatic alcohol has an R-OH structure, wherein R is a saturated or unsaturated, linear or branched monovalent hydrocarbon group having from 4 to 40 carbon atoms, preferably from 6 to 30 carbon atoms, more preferably from 12 to 20 carbon atoms. Examples of the aliphatic alcohols include an alkyl group and an alkenyl group which have 12 to 20 carbon atoms. R may be substituted with at least one hydroxy group.

Examples of the aliphatic alcohol include lauryl alcohol, cetyl alcohol, stearyl alcohol, isostearyl alcohol, behenyl alcohol, undecylenyl alcohol, myristyl alcohol, octyldodecanol, hexyldecanol and a mixture thereof.

The ester oil is a liquid ester obtained by condensation reaction of a saturated or unsaturated, linear or branched C1-C26 aliphatic monoacid or polyacid with a saturated or unsaturated, linear or branched C1-C26 aliphatic monoalcohol or polyalcohol. The total number of carbon atoms of the ester is preferably 10 or more.

Examples of the ester oil include diisobutyl adipate, diethylhexyl succinate, cetyl 2-ethylhexanoate, glyceryl tri2-ethylhexanoate, hexyldecyl ethylhexanoate, triethylhexanoine, neopentyl glycol diethyl hexanoate, trimethylolpropane triethylhexanate, pentaerythrityl tetraethylhexanoate, neopentyl glycol dicaprate, ethylhexyl isononanoate, isononyl isononanoate, isodecyl isononanoate, isotridecyl isononanoate, hexyl laurate, isopropyl myristate, octyldodecyl myristate, isocetyl myristate, isopropyl palmitate, ethylhexyl palmitate, octyl palmitate, ethyl stearate, octyl stearate, isocetyl stearate, ethyl isostearate, isopropyl isostearate, hexyldecyl isostearate, isostearyl isostearate, glyceryl triisostearate, polyglyceryl triisostearate, trimethylolpropane triisostearate, pentaerythrityl tetraisostearate, ethyl oleate, tri(capryl/capric acid) glyceryl, and octyldodecyl 1,2-stearoyl stearate.

### Water phase

In the present emulsion composition, water, or water and a water-soluble substituent may preferably be in a form of droplets of a diameter of 1 to 300 µm, preferably 1 to 50 µm. If the particle size is larger than the afore-mentioned upper limit, the storage stability of the emulsion may be lower. The particle size may be determined by a laser diffraction/scattering particle size distribution measurement apparatus or a polarizing microscope.

The water phase may comprise water, or water and a water-soluble substituent, as usual. The water phase may also comprise a storage stabilizer of an emulsion. Examples of the storage stabilizers for the emulsion include inorganic salts such as sodium chloride, magnesium dichloride and magnesium sulfate. An organic solvent miscible with water may also be incorporated. For instance, the organic solvent has 2 to 20 carbon atoms, preferably 2 to 10 carbon atoms, more preferably 2 to 6 carbon atoms. Examples the organic solvent include polyols such as glycerol, propylene glycol, butylene glycol, pentylene glycol, hexylene glycol, dipropylene glycol and diethylene glycol; and glycol ethers such as a mono-, di- or tri-propylene glycol alkyl ether and a mono-, di- or tri-ethylene glycol alkyl ether.

Other water-soluble substituent includes active components used in cosmetics, such as anti-inflammatory agents such as dipotassium glycyrrhizinate, β-glycyrrhetinic acid, and yomogi extract; antioxidants such as glucosylrutin; swertia extract; blood circulation promoting agents such as benzyl nicotinate; humectants such as sodium hyaluronate, sodium pyrrolidone carboxylate, calendula officinalis flower extract, scutellaria baicalensis root extract and silacoba extract; hormonal agents such as ethinylestradiol; vitamins such as vitamin C; nutrients such as amino acids, peptides, nucleic acids and derivatives thereof; biologically active peptides, various water-soluble anticancer agents, enzymes and repellents.

Other examples are a lower alcohol such as a polyhydric alcohol and ethanol, a water-soluble polymer, a plant extraction extract, a preservative, a metal sealant, an antioxidant, a colorant, a pH adjuster, and a perfume may be appropriately contained.

### Hydrophobic, spherical sol-gel silica fine particles

The present invention is characterized in that the water-in-oil emulsion composition comprises the specific hydrophobic, spherical sol-gel silica fine particles. That is, the present invention is characterized by the hydrophobic, spherical sol-gel silica fine particles having a volumetric median diameter (d50) of 5 to 1500 nm.

In general, synthetic amorphous silica is roughly classified into a wet-process silica and a dry-process silica. Sol-gel silica fine particles are produced by the sol-gel method and are so-called wet-process silica fine particles. The dry-process silica has a high aggregation property. Therefore, though a primary particle size is obtained by particle size distribution determination in a solution dispersion system, the aggregation is remarkable in the microscopic observation in a powder state, so that the results do not match with each other. On the other hand, the sol-gel silica fine particles have a low aggregation property and a high dispersibility. Therefore, a particle size obtained by the particle size distribution determination in a solution dispersion system and an obtained by microscopic observation in a powder state. The wet-process silica has more surface silanol groups than the dry-process silica. The silanol groups of the present sol-gel silica fine particles are hydrophobized by a method described later. The hydrophobic, spherical sol-gel silica fine particles of the present invention preferably have a hydrophobicity of 10 to 70 and an average circularity of 0.8 to 1.

The hydrophobic, spherical sol-gel silica fine particles have a particle diameter of 5 to 1500 nm, preferably 5 to 1000 nm, more preferably 7 to 800 nm, and particularly preferably 10 to 500 nm. If the particle diameter is smaller than the afore-mentioned lower limit, an emulsion may not be formed in some cases. If the particle diameter is larger than the aforesaid upper limit, an emulsion is not formed, or has a small size of droplets of the aqueous phase is not easily attained, so that storage stability of the emulsion may be poor, which is not preferable. The aforesaid particle diameter is a volumetric median diameter, D50. The volumetric median diameter (D50) is a particle diameter corresponding to the cumulative 50 % when the volume particle size distribution is expressed as a cumulative distribution. The volumetric median diameter may be determined by a laser diffraction/scattering particle size distribution measuring apparatus.

In the present invention, "spherical" means not only truly-spherical but also a sphere that is slightly distorted. The shape of a particle is evaluated by circularity obtained by projecting a particle in two dimensions. The circularity is (circumferential length of a circle having an area equal to the particle projected area)/(circumferential length of a particle).

The term "spherical" in the present invention means, in particular, a sphere having an average circularity in the range of 0.8 to 1. Thus, the hydrophobic spherical sol-gel silica fine particles should have an average circularity of 0.8 to 1, preferably 0.85 to 1, more preferably 0.90 to 1. The circularity is determined from an image obtained by an electronic microscope.

The hydrophobic, spherical sol-gel silica fine particles preferably have a D90/D10 of 3 or less, more preferably 2.9 or less, which is an index of volume particle size distributions. In the determination of the particle size distribution, the particle diameter at 10 % in the cumulative distribution is D10. The particle diameter at 90 % in the cumulative distribution is D90. The D90/D10 of 3 or less, more preferably 2.9 or less, means that the particle size distribution curve is sharp (single). When the particle size distribution curve is sharp, the fine particles arrange with a closest-packing structure at the oil/water interface, which is preferable for increased storage stability of the emulsion.

The hydrophobic, spherical sol-gel silica fine particles preferably have a hydrophobicity of 10 to 70, more preferably 30 to 70, as described above. The hydrophobicity is so-called methanol wettability (MW value). A larger hydrophobicity means that a substance is more hydrophobic. When the MW value is 70 or less, a good water-in-oil emulsion is obtained. If the MW value is less than the afore-mentioned lower limit, a water-in-oil emulsion may not be formed in some cases.

The hydrophobic, spherical sol-gel silica fine particles are obtained by a surface-hydrophobizing treatment on hydrophilic spherical silica fine particles which consist substantially of SiO₂ units, and are preferably obtained by hydrolysis of tetraalkoxysilane, as will be described below. The hydrophobic, spherical sol-gel silica fine particles have a primary particle size of starting hydrophilic spherical sol-gel silica fine particles, do not show aggregation, and have a uniform. The hydrophobic, spherical sol-gel silica fine particles show a low agglomeration property despite their fine size. The hydrophobic spherical sol-gel silica fine particles in a water-in-oil emulsion arrange in a closest-packing structure to form a single layer or multiple layers on the oil/water interface, whereby a good emulsion having high storage stability is provided.

The aforesaid description on the hydrophilic spherical silica fine particles, "consist substantially of SiO₂ units", means that the particles are basically composed of SiO₂ units and have at least a plurality of silanol (Si-OH) groups on their surfaces. Some of the hydrolyzable group of tetraalkoxysilane and/or a partial hydrolysis condensation product thereof as the raw material may not be converted into a silanol group and remain, as it is, on or inside the particle surface. These silanol groups and hydrolyzable groups are hydrophobized by the afore-mentioned surface treatment.

The hydrophobic, spherical sol-gel silica fine particles according to the present invention have a group obtained by a reaction of a part or all of the -SiOR' group of the hydrophilic spherical sol-gel silica fine particles with a trifunctional, silane R²Si(OR¹)₃. Here, R' is a hydrogen atom or a substituted or unsubstituted monovalent hydrocarbon group having 1 to 20 carbon atoms. R¹ and R² are, independently of each other, a substituted or unsubstituted monovalent hydrocarbon group having 1 to 20 carbon atoms. R²SiO_{3/2}- bonds to the -SiOR' group present on the surface of hydrophilic spherical sol-gel silica particles by the hydrophobic treatment.

The substituted or unsubstituted monovalent hydrocarbon group having 1 to 20 carbon atoms is preferably a monovalent hydrocarbon group having 1 to 6 carbon atoms, more preferably 1 to 3 carbon atoms, and particularly preferably 1 to 2 carbon atoms. Examples of the monovalent hydrocarbon groups include alkyl groups such as a methyl, ethyl, propyl, isopropyl, butyl and hexyl groups, preferably a methyl, ethyl, propyl and isopropyl groups. Particularly, a methyl and ethyl groups are preferred. In addition, a part or all of the hydrogen atoms of these monovalent hydrocarbon groups may be substituted with halogen atoms such as a fluorine atom, a chlorine atom and a bromine atom, and preferably a fluorine atom.

After the hydrophobic treatment with the trifunctional silane, R²Si(OR¹)₃, the silanol group and the hydrolyzable group present on the surface are preferably hydrophobilized. That is, in a more preferred embodiment of the present hydrophobic, spherical sol-gel silica fine particles, component (c), a silanol group and a hydrolyzable group present on the surface of the hydrophobic, spherical sol-gel silica fine particles react with a silazane compound or a monofunctional silane to form R³₃SiO_{1/2}-. Here, R' is a hydrogen atom or a substituted or unsubstituted, monovalent hydrocarbon group having 1 to 20 carbon atoms, R¹ and R² are, independently of each other, a substituted or unsubstituted, monovalent hydrocarbon group having 1 to 20 carbon atoms, and R³ is a substituted or unsubstituted, monovalent hydrocarbon group having 1 to 10 carbon atoms. On account of the hydrophobization, R³₃SiO_{1/2}- bonds to the silanol group of hydrophilic spherical silica fine particles and the -SiOR¹ group of the trifunctional silane R²Si(OR¹)₃.

In the formula, R³ is, independently of each other, a substituted or unsubstituted, monovalent hydrocarbon group having 1 to 10 carbon atoms, preferably 1 to 6 carbon atoms, particularly preferably 1 or 2 carbon atoms. Examples of R³ include alkyl groups such as methyl, ethyl, propyl, isopropyl, butyl, hexyl, and octyl groups, preferably methyl, ethyl and propyl groups. Particularly, a methyl group and an ethyl group are preferred. A part or all of the hydrogen atoms of the monovalent hydrocarbon group may be substituted with halogen atoms such as a fluorine atom, a chlorine atom and a bromine atom, preferably a fluorine atom.

The present hydrophobic spherical sol-gel silica fine particles are obtained by subjecting the surface of spherical, silica fine particles prepared by a sol-gel method to the hydrophobic treatment, as described above. The preparation may be carried out any known method. The sol-gel method is such that a tetrafunctional silane compound is added to a mixture of a hydrophilic organic solvent such as an alcohol, water and a basic substance and allowed to react to obtain a solvent-dispersion containing hydrophilic spherical silica fine particles and, then, the dispersion is dried to obtain powder. The method will be described in more detail below.

### Preparation method (A)

The hydrophobic, spherical sol-gel silica particles of the present invention are obtained by a process including the following steps:
step (A1): a synthesis step of hydrophilic spherical sol-gel silica fine particles,
step (A2): a surface treatment step with a trifunctional silane compound, and
optional step (A3): a surface treatment step with a monofunctional silane compound.

The steps will be explained in order in further detail below.

### Step (A1): Synthesis step of hydrophilic spherical sol-gel silica fine particles

The hydrophilic spherical sol-gel silica fine particles are obtained by a hydrolysis and condensation of a tetrafunctional silane compound represented by the general formula (I):

Si(OR¹)₄ (I)

and/or a partial hydrolysate thereof. In the formula (I), R¹ is a substituted or unsubstituted, monovalent hydrocarbon group having 1 to 20 carbon atoms. The hydrolysis and condensation reaction may be carried out according to a conventional manner. For example, the reaction temperature may be between 20 to 70 degrees C. For instance, the reaction is carried out by adding a tetrafunctional silane compound and/or a partial hydrolysate thereof to a mixture of water, a hydrophilic organic solvent and a basic substance to obtain a solvent-dispersion comprising hydrophilic, spherical silica fine particles consisting essentially of SiO₂ units.

Examples of the tetrafunctional silane compound represented by the general formula (1) include tetraalkoxysilanes such as tetramethoxysilane, tetraethoxysilane, tetrapropoxysilane and tetrabutoxysilane; and tetraphenoxysilanes. Preferred are tetramethoxysilane, tetraethoxysilane, tetrapropoxysilane and tetrabutoxysilane. Particularly, tetramethoxysilane and tetraethoxysilane are preferred. Examples of the partial hydrolysis condensation product of the tetrafunctional silane compound represented by the general formula (I) include a methylsilicate and an ethyl silicate.

The hydrophilic organic solvent may be not particularly limited as long as it dissolves the afore-mentioned tetrafunctional silane compound, a partially hydrolyzed condensate and water. For example, alcohols, cellosolves such as methyl cellosolve, ethyl cellosolve, butyl cellosolve and cellosolve acetate, ketones such as acetone and methyl ethyl ketone, and ethers such as dioxane and tetrahydrofuran may be used. Preferred are alcohols and cellosolves, particularly alcohols.

The alcohol is, for example, represented by the formula (V):

R⁴OH (V),

wherein R⁴ is a monovalent hydrocarbon group having 1 to 6 carbon atoms, preferably 1 to 4 carbon atoms, particularly preferably 1 to 2 carbon atoms. Examples of the monovalent hydrocarbon group includes alkyl groups such as a methyl, ethyl, propyl, isopropyl and butyl groups, preferably a methyl, ethyl, propyl and isopropyl groups. A methyl and an ethyl group are more preferable. Examples of the alcohol include methanol, ethanol, propanol, isopropanol and butanol. Methanol and ethanol are more preferable.

Examples of the basic material include ammonia, dimethylamine and diethylamine, preferably ammonia and diethylamine. Particularly, ammonia is preferable. A desired amount of a basic material may be dissolved in water, which is then, mixed with the hydrophilic organic solvent.

The amount of water to be mixed with the basic material is preferably 0.5 to 5 mol, more preferably 0.6 to 2 mols, and particularly preferably 0.7 to 1 mol, per total mole of the alkoxy group of the tetrafunctional silane compound and the partial hydrolysate thereof. A mass ratio of the amount of the hydrophilic organic solvent to the amount of water is preferably 0.5 to 10, more preferably 3 to 9, and particularly preferably from 5 to 8. The amount of the basic material is preferably 0.01 to 2 mol, more preferably from 0.02 to 0.5 mol, and particularly preferably from 0.04 to 0.12 mol, per total mole of the alkoxy group of the tetrafunctional silane compound and the partial hydrolysate thereof.

The solvent dispersion of hydrophilic, spherical sol-gel silica fine particles is obtained in step (A1). The concentration of the spherical sol-gel silica fine particles in the dispersion is usually 3 % by mass or more to less than 15 % by mass, and preferably 5 to 10 % by mass. If the concentration of the silica fine particles is too low, the productivity is lower, which is not preferable. If the concentration is too high, aggregation among the spherical silica fine particles may occur.

### Step (A2): Surface treatment with a trifunctional silane compound

A trifunctional silane compound represented by the general formula (II):

R²Si(OR¹)₃ (II)

and/or a partial hydrolysate thereof is added to the solvent dispersion of the hydrophilic spherical silica fine particles obtained in the aforesaid step (A1) and, then, allowed to react with a silanol group or an alkoxy group present on the surface of the hydrophilic spherical silica fine particle to obtain a solvent dispersion of first hydrophobic spherical sol-gel silica fine particles on the surface of which the R²SiO_{3/2} unit is introduced. In the formula (II), R¹ and R² are as defined above. The hydrophilic spherical silica fine particles obtained in the aforesaid step (A1) tend to aggregate. The aggregation among the silica fine particles is suppressed by the hydrophobizing treatment in step (A2).

Examples of the trifunctional silane compound include trialkoxysilanes such as methyltrimethoxysilane, methyltriethoxysilane, ethyltrimethoxysilane, ethyltriethoxysilane, *n*-propyltrimethoxysilane, n-propyltriethoxysilane, isopropyltrimethoxysilane, isopropyltriethoxysilane, butyltrimethoxysilane, butyltriethoxysilane, hexyltrimethoxysilane, hexyltriethoxysilane, trifluoropropyltrimethoxysilane, trifluoropropyltriethoxysilane, heptadecafluorodecyltrimethoxysilane and heptadecafluorodesyltriethoxysilane. Methyltriethoxysilane and ethyltriethoxysilane are preferred, and methyltriethoxysilane is more preferred. The partially hydrolyzed condensates thereof may also be used.

The amount of the trifunctional silane compound is 0.001 to 1 mol, preferably 0.01 to 0.1 mol, and particularly preferably 0.01 to 0.05 mol, per mole of the Si atoms of the hydrophilic spherical silica fine particles. If the amount is less than 0.01 mol, the dispersibility may be worse. If the amount is more than 1 mol, agglomeration of the spherical silica fine particles among the particles may occur. It is noted that 1 mole of the Si atoms of the hydrophilic spherical silica fine particles means such with the supposition that all of tetraalkoxysilane reacted to give the hydrophilic particles.

The solvent dispersion of the first hydrophobic spherical sol-gel silica fine particles is obtained in the aforesaid step (A2). The concentration of the spherical silica fine particles in the dispersion is preferably 3 % by mass and more and less than 15 % by mass, and preferably 5 to 10 % by mass. If the concentration of the spherical silica fine particles is too low, the productivity is lower, which is not preferable. If the concentration is too high, there is a possibility that agglomeration of the spherical silica fine particles occur. The first hydrophobic spherical sol-gel silica fine particles are converted into a powder state by atmospheric pressure drying or vacuum drying.

### Step (A3): Surface treatment with a monofunctional silane compound

More preferably, a silazane compound represented by the following general formula (III) and/or a monofunctional silane compound represented by the following general formula (IV) are added to the solvent dispersion of the first hydrophobic spherical sol-gel silica fine particles obtained in the aforesaid step (A2) and allowed to react with the silanol group or the alkoxy group present on the surface of the fine particles to obtain a solvent dispersion of second hydrophobic spherical sol gel silica fine particles in which the R³₃SiO_{1/2} unit is further introduced on the surface of the first hydrophobic, spherical sol gel silica fine particle.

R³₃SiNHSiR³₃ (III)

wherein R³ is as defined above.

R³₃SiX (IV)

wherein R³ is as defined above and X is a hydroxy group or a hydrizable group.

In step (A3), R³₃SiO_{1/2} unit is introduced on the surface of the hydrophobic spherical sol-gel silica particles, hereinafter the particles are referred to as second hydrophobic spherical sol-gel silica particles.

Examples of the hydrizable group represented by X include alkoxy groups, amino groups, acyloxy groups and a chlorine atom, preferably, alkoxy groups and amino groups. Among the alkoxy groups, particularly a methoxy group and an ethoxy group are preferred.

Examples of the silazane compound represented by the general formula (III) include hexamethyldisilazane, hexaethyldisilazane, and dioctyltetramethyldisilazane, and preferably hexamethyldisilazane. Examples of the monofunctional silane compound represented by the general formula (IV) include monosilanol compounds such as trimethylsilanol and triethylsilanol; monochlorosilanes such as trimethylchlorosilane and triethylchlorosilane; monoalkoxysilanes such as trimethylmethoxysilane and trimethylethoxysilane, monoaminosilanes such as trimethylsilyldimethylamine and trimethylsilyldiethylamine; and monoacyloxysilanes such as trimethylacetoxysilane, preferably, trimethylsilanol, trimethylethoxysilane and trimethylsilyldiethylamine. Trimethylsilanol and trimethylethoxysilane are particularly preferable.

The amount of the compounds represented by the above formula (III) or (IV) is 0.01 to 30 moles, preferably 0.05 to 20 moles, per mole of the Si atoms of the hydrophilic spherical silica fine particles. If the amount is less than 0.01 mole, agglomeration of the particles taken out is much noticeable and, thereby, the particles may not be successfully dried and separated, which is not preferable. An amount of more than 30 moles is economically disadvantageous. The hydrophobicity of the spherical silica fine particles to be taken out is arbitrarily controlled by the amount of the compounds represented by the formula (III) or (IV) in the hydrophobicity treatment.

The solvent dispersion of the second hydrophobic spherical sol-gel silica fine particles is obtained in the aforesaid step (A3). The concentration of the spherical silica fine particles in the dispersion is preferably from 3 % by mass to less than 15 % by mass, preferably 5 to 10 % by mass. If the concentration of the spherical silica fine particles is too low, the productivity is lowers which is not preferable. If the concentration is too high, there is a possibility that agglomeration of spherical silica fine particles occurs. The hydrophobic spherical sol-gel silica fine particles are converted into a powder state by atmospheric pressure drying or vacuum drying.

### Water-in-oil emulsion composition

The present emulsion composition is a water-in-oil emulsion composition comprising the oily substance, the water phase substance, and the hydrophobic, spherical sol-gel silica fine particles. The amount of the spherical sol-gel silica fine particles in the emulsion composition is preferably from 0.5 to 40 parts by mass, more preferably from 7 to 25 parts by mass, relative to 100 parts by mass of the oily substance. If the amount of the spherical silica fine particles is less than the lower limit or more than the upper limit, a stable emulsion composition is not be obtained.

The amount of the water phase substance is preferably from 11 to 800 parts by mass, more preferably from 100 to 500 parts by mass, based on 100 parts by mass of the oily substance. If the amount is less than the lower limit or more than the upper limit, the viscosity of the emulsion composition is too low, and the storage stability may be poor. A surfactant may be added into the emulsion composition to enhance storage stability. However, the amount of the surfactant is preferable minimized or no surfactant is added, because of safety concerns in use of cosmetics and skin preparations. Preferably, the emulsion composition of the present invention does not contain a surfactant.

The method for preparing the water-in-oil emulsion composition is not particularly limited, and may be any known method for preparing an emulsion. It is preferable that the oily substance and the hydrophobic spherical sol-gel silica fine particles are mixed and, then, the water phase substance is gradually added to the mixture to emulsify. An emulsion having an excellent storage stability is efficiently obtained by mixing the oily substance and the hydrophobic spherical sol-gel silica fine particles in advance as mentioned above. The water phase substance and the hydrophobic, spherical sol-gel silica fine particles have low affinity and low miscibility. Therefore, if the water phase substance and the hydrophobic spherical sol-gel silica fine particles are mixed first, the emulsifying ability of the fine particles may be lower, and a good emulsion may not be obtained, which is not preferable. When a water-soluble material is incorporation, such is preferably dissolved in water in advance.

The method for mixing the oily substance and the hydrophobic, spherical sol-gel silica fine particles is not particularly limited. Use may be made of conventional agitators such as propeller blades, turbine blades and paddle blades, and emulsifying-dispersion machines such as high-speed rotary centrifugal radiation type agitators such as homodispers and high-speed rotating shear type agitators such as homomixers.

The method for adding the water phase substance to the mixture of the oily substance and the hydrophobic spherical sol-gel silica fine particles is not particularly limited. For instance, the water phase substance is added to the mixture and then stirred, or the water phase substance is added at one time or gradually while stirring the mixture. When the water phase substances are added gradually, the addition may be continuous or stepwise. The stirring may be conducted with an emulsifying-dispersion machine such as a high-speed rotary centrifugal radiation type stirrer such as a homodisper or a high-speed rotating shear type stirrer such as a homomixer. In order to improve the storage stability, the obtained water-in-oil emulsion composition may be further treated by an emulsifying machine such as a high-pressure jet emulsifying and dispersing machine such as a pressure type homogenizer, a colloid mill, or an ultrasonic emulsifying machine.

The water-in-oil emulsion composition of the present invention has excellent storage stability, and is particularly useful for cosmetics and skin preparations. Examples of the cosmetics include skin cosmetics such as a milky lotion, a cream, a foundation, a cosmetic base, a concealer, a lip cream, a blush, an eye shadow and a mascara. Examples of the skin preparations include a topical cream and a formentation.

### Examples

The present invention will be explained below in further detail with reference to a series of the Examples and the Comparative Examples. However, the present invention is in no way limited by these Examples.

### Synthesis of hydrophobic spherical sol-gel silica fine particles

### Synthesis Example 1

### Step (A1): Synthesis process of hydrophilic spherical sol-gel silica fine particles

To a three-liter glass reactor equipped with a stirrer, a dropping funnel and a thermometer, were placed and mixed 217.0 g of ethanol, 2.3 g of water and 17.3 g of 28 % aqueous ammonia to obtain a solution. This solution was heated to 60 degrees C, and 547.0 g (2.63 mol) of tetraethoxysilane and a mixed solution of 109.8 g of water and 27.5 g of 28 % aqueous ammonia were added dropwise to the solution over 3 hours with stirring. After the dropwise addition, the mixture was allowed to hydrolysis for further 0.5 hour with stirring to obtain a solvent dispersion of hydrophilic spherical sol-gel silica fine particles having an -SiOR' group. R' is substantially mostly a hydrogen atom and may contain a small amount of an ethoxy group.

### Step (A2): Surface treatment with a trifunctional silane compound

To the dispersion of hydrophilic, spherical sol-gel silica fine particles obtained in step (A1), was added dropwise 4.7 g (0.03 mol) of methyltriethoxysilane for 0.5 hour at room temperature. The mixture was continuously stirred for further 12 hours after the dropwise addition to obtain a dispersion of spherical silica particles in which a part of the silanol groups present on the surface of the silica fine particles was hydrophobized, hereinafter referred to as a dispersion of first hydrophobic, spherical sol-gel silica fine particles.

### Step (A3): Surface treatment with a monofunctional silane compound

To the dispersion of first hydrophobic, spherical sol-gel silica fine particles obtained in step (A2), was added 84.5 g (0.52 mol) of hexamethyldisilazane at room temperature to attain a dispersion. The dispersion was heated to a temperature within 50 to 60 degrees C and allowed to react for 6 hours so as to trimethylsilylate the silanol groups present on the surface of the spherical silica particles. Next, the solvent in this dispersion was distilled off at a reduced pressure of 6650 Pa and 130 degrees C to obtain 168 g of spherical sol-gel silica fine particles **1,** hereinafter referred to as second hydrophobic, spherical sol-gel silica fine particles. The average circularity determined as will be described later was 0.87, which meant "spherical".

### Synthesis Example 2

Steps (A1) through (A3) in Synthesis Example 1 were repeated, except that the reaction temperature, 60 degrees C, in step (A1) was changed to 50 degrees to obtain 166 g of spherical sol-gel silica fine particles **2.**

### Synthesis Example 3

Steps (A1) through (A3) in Synthesis Example 1 were repeated, except that the reaction temperature, 60 degrees C, in step (A1) was changed to 40 degrees C to obtain 165 g of spherical sol-gel silica fine particles **3.**

### Synthesis Example 4

Steps (A1) through (A3) in Synthesis Example 1 were repeated, except that the reaction temperature, 60 degrees C, in step (A1) was changed to 70 degrees C to obtain 170 g of spherical sol-gel silica fine particles **4.**

### Synthesis Example 5

### Step (A1): Synthesis of hydrophilic spherical silica fine particles

To a three-liter glass reactor equipped with a stirrer, a dropping funnel, and a thermometer, were placed and mixed 793.0 g of methanol, 32.1 g of water, and 40.6 g of 28 % aqueous ammonia to obtain a solution. This solution was adjusted to 30 degrees C. 646.5 grams (4.25 mol) of tetramethoxysilane and a mixed solution of 129.1 g of water and 31.8 g of 28 % aqueous ammonia were added dropwise to the mixture over 3 hours with stirring. After the dropwise addition, the mixture was allowed to hydrolysis for further 0.5 hour with stirring to obtain a solvent dispersion of hydrophilic spherical sol-gel silica fine particles having an -SiOR' group. R' is substantially mostly a hydrogen atom and may contain a small amount of methoxy groups.

### Step (A2): Surface treatment with a trifunctional silane compound

To the dispersion of hydrophilic, spherical sol-gel silica fine particles obtained in step (A1), was added dropwise 5.8 g (0.04 mol) of methyltrimethoxysilane for 0.5 hour at room temperature. The mixture was continuously stirred for further 12 hours after the dropwise addition to obtain a dispersion of spherical silica particles in which a part of the silanol groups present on the surface of the silica fine particles was hydrophobized, hereinafter referred to as a dispersion of first hydrophobic, spherical sol-gel silica fine particles.

### Step (A3): Surface treatment with a monofunctional silane compound

To the dispersion of first hydrophobic, spherical sol-gel silica fine particles obtained in step (A2), was added 136.9 g (0.85 mol) of hexamethyldisilazane at room temperature to attain dispersion. The dispersion was heated to a temperature within 50 to 60 degrees C and allowed to react for 6 hours so as to trimethylsilylate the silanol groups present on the surface of the spherical silica particles. Next, the solvent in this dispersion was distilled off at a reduced pressure of 6650 Pa and 130 degrees C to obtain 285 g of spherical sol-gel silica fine particles **5,** hereinafter referred to as second hydrophobic, spherical sol-gel silica fine particles. The average circularity determined as will be described later was 0.90, which meant "spherical".

### Synthesis Example 6

Steps (A1) through (A3) of Synthesis Example 5 were repeated, except that the amount of hexamethyldisilazane, 136.9 g, in step (A3) of Synthesis Example 5 was changed to 34.2 g to obtain 280 g of spherical sol-gel silica fine particles **6.**

### Comparative synthesis example 7

Steps (A1) through (A3) of Synthesis Example 5 were repeated, except that the amount of hexamethyldisilazane, 136.9 g, in step (A3) of Synthesis Example 5 was changed to 13.6 g to obtain 281 g of spherical sol-gel silica fine particles **7.**

The particle size of each of the hydrophilic spherical sol-gel silica fine particles obtained each in step (A1) was determined according to the following manners. The particle size and the particle size distribution of the spherical sol-gel silica particles **1** to **7** obtained in Synthesis Examples 1 to 7 were determined according to the following manners.

### 1. Determination of the particle size of the hydrophilic, spherical silica fine particles obtained in step (A1)

The dispersion of the hydrophilic spherical sol-gel silica fine particles obtained in step (A1) was added to methanol so as to attain a concentration of the silica fine particles of 0.5 mass %, and the silica fine particles were dispersed by ultrasonication for 10 minutes. The particle size distribution of the dispersed silica fine particles was determined by a dynamic light scattering method/laser doppler method with a nanotrack partical size distribution analyzers (ex Nikkiso Co., Ltd., trade name: UPA-EX150). A volumetric median diameter was calculated. A median diameter is a particle diameter at 50% in cumulative particle size distribution. The results are as shown in Table 1.

### 2. Particle size determination of the spherical sol-gel silica fine particles obtained in step (A3) and a determination of particle size distribution, D90/D10,

Each of the spherical sol-gel silica particles 1 to 7 obtained in step (A3) was added to methanol so as to attain a concentration of the silica fine particles of 0.5 mass %, and the silica particles were dispersed by ultrasonication for 10 minutes. The particle size distribution of the dispersed silica fine particles was determined by a dynamic light scattering method/laser doppler method with a nanotrack partical size distribution analyzers (ex Nikkiso Co., Ltd., trade name: UPA-EX150). The volumetric median diameter was calculated. The results are as shown in Table 1.

In the determination of the particle size distribution D90 / D10, the particle diameter at 10 % in the cumulative distribution is D10. The particle diameter at 90 % is D90. D90 / D10 is the particle size distribution here. The results are as shown in Table 1.

### 3. Observation of a shape and form of the spherical sol-gel silica fine particles obtained in step (A3)

A shape of the spherical sol-gel silica fine particles were observed by an electron microscope (ex Hitachi, Ltd., trade name: S-4700, magnification: × 100,000). The shape of the fine particle is evaluated by a circularity of the particle projected in two-dimension.

Circularity is (circumferential length of a circle having an area equal to the particle projected area) / (circumferential length of a particle).

Particles having an average circularity of 0.8 to 1 are called as "spherical". The results are as shown in Table 1.

### 4. Determination of a hydrophobicity of the spherical sol-gel silica fine particles obtained in step (A3)

50 ml of water was poured into a 200-milliliter beaker, and 0.2 g of spherical sol-gel silica fine particles was placed in the beaker. Methanol was added dropwise in the beaker by a burette with stirring by a magnetic stirrer. When all of the silica fine particles were made wet by the liquid present in the beaker, the dropwise addition was stopped. Here, methanol was added into the liquid through a tube so as not to directly contact the silica fine particles. The volume % of the methanol in the methanol-water mixed solvent is defined as hydrophobicity. The silica fine particles obtained in Comparative Synthesis Example 7 had a hydrophobicity of 0. Therefore, the spherical sol-gel silica fine particles obtained in Comparative Synthesis Example 7 were hydrophilic. In contrast, the spherical sol-gel silica particles obtained in Synthesis Examples 1 to 6 had a hydrophobicity of 33 to 67 as shown in Table 1 and, thus, were hydrophobic. Hereinafter, these are referred to as "hydrophobic, spherical sol-gel silica fine particles".

**Table 1**

| | Synthesis Example 1 | Synthesis Example 2 | Synthesis Example 3 | Synthesis Example 4 | Synthesis Example 5 | Synthesis Example 6 | Comparative Synthesis Example 7 |
|---|---|---|---|---|---|---|---|
| Spherical sol-gel silica fine particles | 1 | 2 | 3 | 4 | 5 | 6 | 7 |
| Volumetric median diameter of the hydrophilic spherical silica fine particles, nm | 240 | 350 | 1000 | 160 | 70 | 70 | 70 |
| Volumetric median diameter of the (hydrophobic) spherical sol-gel silica fine particles, nm | 240 | 350 | 1000 | 160 | 70 | 70 | 70 |
| Hydrophobicity | 67 | 67 | 67 | 67 | 58 | 33 | 0 |
| Distribution, D90/D10 | 2.20 | 2.30 | 2.25 | 2.30 | 3.0 | 3.0 | 3.0 |
| Shape | Spherical | Spherical | Spherical | Spherical | Spherical | Spherical | Spherical |
| Average circularity | 0.87 | 0.89 | 0.90 | 0.88 | 0.90 | 0.90 | 0.90 |

### Examples 1-11, and Comparative Examples 1-4

The oily substance used in the following Examples and Comparative Examples are as follows.
Oily substance 1: KF-96A-6cs (polydimethylsiloxane having a kinematic viscosity of 6mm2/s, ex Shin-Etsu Chemical Co., Ltd.)
Oily substance 2: KF-995 (decamethylcyclopentasiloxane, ex Shin-Etsu Chemical Co., Ltd.)
Oily substance 3: TMF-1.5 (methyltrimethicone, ex Shin-Etsu Chemical Co., Ltd.)
Oily substance 4: liquid paraffin (ex Wako Pure Chemical Industries, Ltd.)
Oily substance 5: Clairol (hydrocarbon oil, ex Sonneborn Corporation)
Oily substance 6: SALACOS 99 (isononyl isononate, ex Nisshin Oilio Co., Ltd.)

In Comparative Example 2, VP NX-2000 (dry silica, ex Nippon Aerosil Co., Ltd.) was used as solid fine particles. VP NX-2000 has a volumetric median diameter of 1.3 µm, determined by a particle size distribution analyzer, MICRO TRACK, ex Nikkiso Co., Ltd. and a degree of hydrophobicity of 9.09 (wettability by methanol).

### Preparation of water-in-oil emulsion composition

An oily substance and (hydrophobic) spherical sol-gel silica fine particles were placed in a 200-milliliter plastic container in the compositions as shown in Tables 2 and 3 below. Then, those were stirred at a rotation speed of 4000 to 5000 rpm by a homomixer, (*i.e*. emulsifying and dispersing apparatus, LABOLUTION (A), ex PRIMIX Corporation) which was a high-speed rotary shear-type stirrer. With stirring, water phase substance was gradually added in the amount as shown in Table 2 or 3 and emulsified to obtain a water-in-oil emulsion composition.

The compositions of Comparative Examples 1 and 2 were not emulsified and no emulsion was obtained. Therefore, the following evaluations were not conducted.

In the following Tables 2 and 3, the numeric values in parentheses indicate parts by mass of each component, per 100 parts by mass of the oily substance.

### Determination of particle size and storage stability of the emulsions

An emulsion particle size of the emulsion composition was observed by a polarizing microscope (ECLIPSE LV100POL, ex Nikon Corporation). Each emulsion composition was taken up in a glass bottle and stored at room temperature for one month. Then, the appearance was observed to evaluate the storage stability of the emulsion composition.

The storage stability was rated by the following indices.
A: No separation between the oil phase and the water phase was observed.
B: Partial phase separation occurred.
C: The composition was completely separated into two phases (oil phase/water phase)

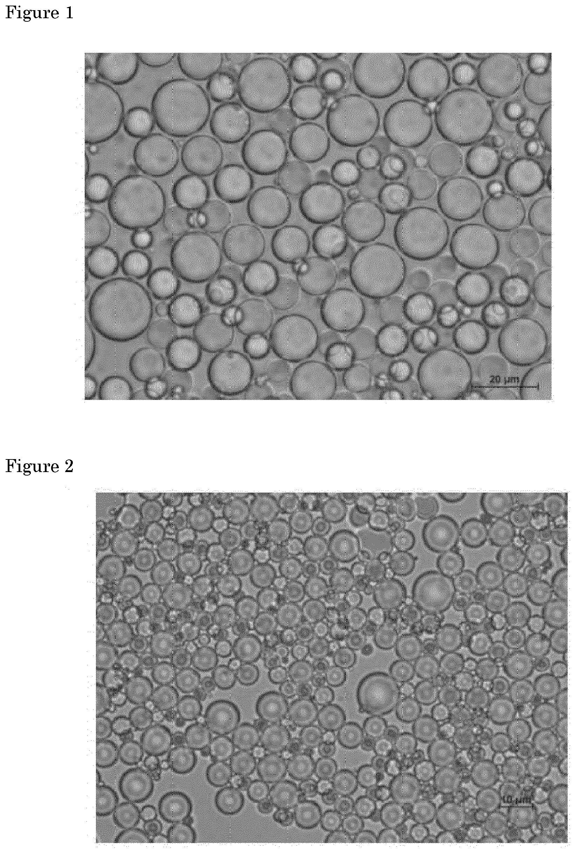

Figures 1 and 2 show the images taken by a polarizing microscope on the emulsion compositions obtained in Examples 4 and 10. The results of the observation by a polarizing microscope are as shown in Figures 1 and 2. Figure 1 is on the emulsion composition of Example 4 and Figure 2 is on the emulsion composition of Example 10.

**Table 2**

| Part by mass | | Example | | | | | | | | | | |
|---|---|---|---|---|---|---|---|---|---|---|---|---|
| | | 1 | 2 | 3 | 4 | 5 | 6 | 7 | 8 | 9 | 10 | 11 |
| Solid particles | Hydrophobic, spherical sol-gel silica fine particle dispersion 2 | 5 (10) | | | 5 (16.7) | 5 (10) | 5 (7.1) | 5 (16.7) | | | | |
| | D50: 350 nm, | | | | | | | | | | | |
| | Average circularity: 0.89, | | | | | | | | | | | |
| | Hydrophobicity: 67 | | | | | | | | | | | |
| | Hydrophobic, spherical sol-gel silica fine particle dispersion 4 | | 5 (10) | | | | | | | | | |
| | D50: 160 nm, | | | | | | | | | | | |
| | Average circularity: 0.88, | | | | | | | | | | | |
| | Hydrophobicity: 67 | | | | | | | | | | | |
| | Hydrophobic spherical sol-gel silica fine particle dispersion 5 | | | 5 (10) | | | | | | | | |
| | D50: 70 nm, | | | | | | | | | | | |
| | Average circularity: 0.90 | | | | | | | | | | | |
| | Hydrophobicity: 58 | | | | | | | | | | | |
| Oil phase | 1: KF-96A-6cs | 50 | 50 | 50 | 30 | 50 | 70 | | | | | |
| | 2: KF-995 | | | | | | | 30 | | | | |
| | 3: TMF-1.5 | | | | | | | | 30 | | | |
| | 4: Liquid paraffin | | | | | | | | | 30 | | |
| | 5: Clairol | | | | | | | | | | 30 | |
| | 6: SALACOS 99 | | | | | | | | | | | 30 |
| Water phase | Ion-exchanged water | 45 (90) | | | 65 (217) | 45 (90) | 25 (35.7) | 65 (217) | | | | |
| Particle size, µm | | 30 | 20 | 10 | 20 | 30 | 30 | 20 | 15 | 10 | 10 | 10 |
| Storage stability | | A | A | A | A | A | A | A | A | A | A | A |

**Table 3**

| Part by mass | | Comparative Example | | | |
|---|---|---|---|---|---|
| | | 1 | 2 | 3 | 4 |
| Solid particles | Hydrophobic spherical sol-gel silica fine particle 2 | | | 5 (50) | 5 (5.6) |
| | Hydrophobic spherical sol-gel silica fine particle 7 | 5 (10) | | | |
| | D50: 70 nm | | | | |
| | Average circularity: 0.90 | | | | |
| | Hydrophobicity: 0 | | | | |
| | Dried silica | | 5 (10) | | |
| Oil phase | 1: KF-96A-6cs | 50 | 50 | 10 | 90 |
| Water phase | Ion-exchanged water | 45 (90) | 45 (90) | 85 (850) | 5 (5.6) |
| Particle size, µm | | Not emulsified. | Not emulsified. | 50 < | 50 < |
| Storage stability | | | | B | B |

As shown in Table 2, each of the water-in-oil emulsion compositions of the present invention (Examples 1 to 11) has a particle size (droplet size) in 10 to 30µm and the high storage stability.

In contrast, the composition of Comparative Example 1 comprising spherical sol-gel silica fine particles having a hydrophobicity of 0, and the composition of Comparative Example 2 comprising dry silica, as solid fine particles, having a hydrophobicity of 10 or less were not emulsified. The compositions of Comparative Examples 3 and 4, in which the amount of the water phase is too small or too large were emulsified, but had low storage stability.

### Evaluation of an adsorption of the hydrophobic, spherical sol-gel silica fine particles at the oil/water interface of the water-in-oil emulsion composition

The adsorption of the silica fine particles at the oil/water interface of the present water-in-oil emulsion composition was observed by an electronic microscope (S-4700, scanning microscope, ex Hitachi High Technologies, Ltd.) and evaluated.

In advance to the aforesaid observation, it was necessary to immobilize that the emulsion composition. To this end, the procedure in Example 4 were repeated to prepare a water-in-oil emulsion composition, except that KF-96A-6cs in Example 4 was changed to a reaction curable silicone composition comprising a polyorganosiloxane having a monovalent olefin unsaturated group and an organohydrogenpolysiloxane. Then, a platinum catalyst was added and the oil phase was cured to prepare a sample.

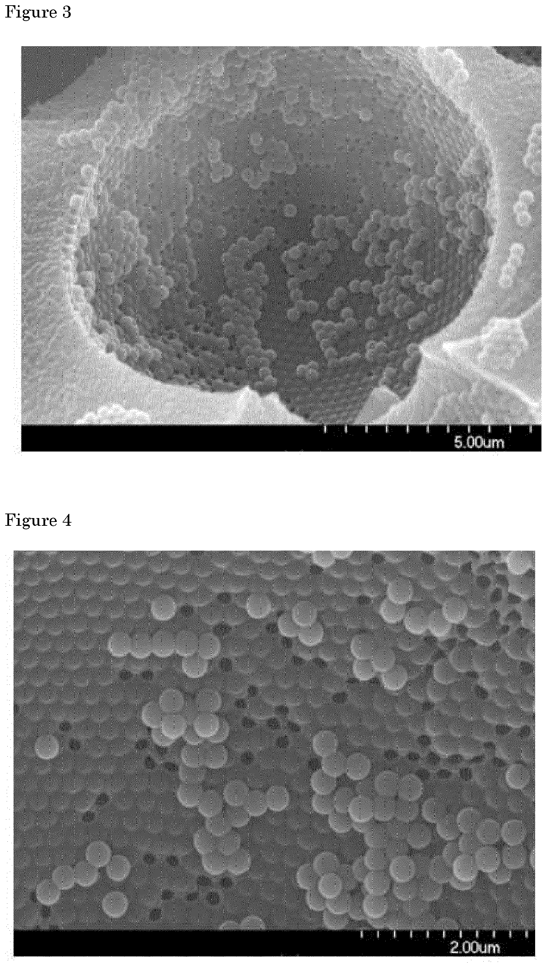

The sample was cut with a surgical knife and the water was evaporated at 105 degrees C in an air thermostat. Figures 3 and 4 show images taken by the electron microscope. Many depressions are seen, where water contained in the emulsion composition is used to exist. Figure 3 is an overall view of the particles. Figure 4 is a partial view of a particle.

As shown in Figures 3 and 4, the hydrophobic, spherical sol-gel silica fine particles which are solid are regularly arranged and adsorbed in a closest-packing at the oil/water interface in the present water-in-oil emulsion composition.

The silica fine particles form a layered structure at the oil/water interface. Thus, the interface membrane is of one layer or, even, multi-layers.

That is, on account of the hydrophobic, spherical sol-gel silica fine particles being regularly arranged at the oil/water interface is closest-packing, and on account of the high adsorption force of the sol-gel silica fine particles, the interface membrane is strengthened to provide a stable emulsion.

### Industrial Applicability

The water-in-oil emulsion composition of the present invention has excellent storage stability and is particularly useful for cosmetics and skin preparations.

## Claims

1. A water-in-oil emulsion composition comprising
(a) an oily substance in an amount of 100 parts by mass,
(b) water, or water and a water-soluble substance in an amount of 11 to 800 parts by mass, and
(c) hydrophobic, spherical sol-gel silica fine particles having a volumetric median diameter (D50) of 5 to 1500 nm in an amount of 0.5 to 40 parts by mass.

2. The water-in-oil emulsion composition according to claim 1, wherein the hydrophobic, spherical sol-gel silica fine particles have a hydrophobicity degree of 10 to 70.

3. The water-in-oil emulsion composition according to claim 1 or 2, wherein the hydrophobic, spherical sol-gel silica fine particles have an average circularity of 0.8 to 1.

4. The water-in-oil emulsion composition according to any one of claims 1 to 3, wherein component (c) has a group formed by reaction of a part or all of a -SiOR' group of a hydrophilic spherical sol-gel silica fine particle with a trifunctional silane, R²Si(OR¹)₃, wherein R' is a hydrogen atom or a substituted or unsubstituted monovalent hydrocarbon group having 1 to 20 carbon atoms, and R¹ and R² are, independently of each other, a substituted or unsubstituted monovalent hydrocarbon group having 1 to 20 carbon atoms.

5. The water-in-oil emulsion composition according to claim 4, wherein component (c) is hydrophobic spherical sol-gel silica fine particles, wherein an R³₃SiO_{1/2}- group formed by reaction of a -SiOR' group present on the surface of the hydrophobic, spherical sol-gel silica fine particle with a silazane compound or with a monofunctional silane, wherein R' is as defined above, and R³ is a substituted or unsubstituted, monovalent hydrocarbon group having 1 to 10 carbon atoms.

6. The water-in-oil emulsion composition according to any one of claims 1 to 5, wherein the oily substance (a) is at least one selected from silicone oils, hydrocarbon oils and fatty alcohols.

7. The water-in-oil emulsion composition according to any one of claims 1 to 6, wherein the hydrophobic, spherical sol-gel silica fine particles (c) have a volumetric median diameter (D50) of 5 to 1000 nm.

8. The water-in-oil emulsion composition according to any one of claims 1 to 7, wherein the hydrophobic, spherical sol-gel silica fine particles (c) have a Volumetric particle size distribution, D90 / D10, of 3 or less.

9. The water-in-oil emulsion composition according to any one of claims 1 to 8, containing no surfactant.
